# EUROPEAN PATENT SPECIFICATION

(11) **EP 4 206 208 B1**
(45) Date of publication and mention of the grant of the patent: **09.04.2025**
(21) Application number: 20967796.2
(22) Date of filing: 31.12.2020
(51) Int. Cl.: C07F 7/22, C07H 1/00, C07H 13/08, C07H 23/00, B01J 31/12

(54) **USE OF MONO-TIN ORGANIC COMPOUNDS**
VERWENDUNG VON ORGANISCHEN MONOZINNVERBINDUNGEN
UTILISATION DE COMPOSÉS ORGANIQUES DE MONO-ÉTAIN

(43) Date of publication of application: 05.07.2023
(73) Proprietor: Anhui Jinhe Industrial Co., Ltd., Chuzhou, Anhui 239200 (CN)
(72) Inventor: TAO, Changwen, Chuzhou City, Anhui 239200 (CN); ZHAO, Jingang, Chuzhou City, Anhui 239200 (CN); ZHENG, Xuelian, Chuzhou City, Anhui 239200 (CN); LUO, Jialong, Chuzhou City, Anhui 239200 (CN); CHEN, Chaohui, Chuzhou City, Anhui 239200 (CN)
(74) Representative: Schneiders & Behrendt Bochum
(86) International application number: PCT/CN2020/142289
(87) International publication number: WO 2022/141457

(56) References cited:
- CN-A- 102 453 237
- GB-A- 1 593 589
- GB-A- 797 073
- US-A- 3 361 775
- U. WUTHIER, ET AL.: "Anion selectivities of triorganyltin acetates and halides in solvent polymeric membranes", HELVETICA CHIMICA ACTA, vol. 69, no. 6, 10 September 1986 (1986-09-10), Verlag Helvetica Chimica Acta, Basel, CH, pages 1435 - 1441, XP055948449, ISSN: 0018-019X, DOI: 10.1002/hlca.19860690616
- D.W. ALLEN, ET AL.: "The preparation and tin-119m Mössbauer spectra of tri-n-butylstannyl esters of heterocyclic and substituted aromatic carboxylic acids", JOURNAL OF ORGANOMETALLIC CHEMISTRY, vol. 156, no. 2, 22 August 1978 (1978-08-22), Elsevier-Sequoia, Lausanne, CH, pages 359 - 368, XP093118551, ISSN: 0022-328X, DOI: 10.1016/S0022-328X(00)93542-3
- HUNG-VIET PHAM, ET AL.: "The coupling 2J(119Sn-O-13CO) in tributyltin acetate", JOURNAL OF ORGANOMETALLIC CHEMISTRY, vol. 403, no. 3, 19 February 1991 (1991-02-19), Elsevier-Sequoia, Lausanne, CH, pages 311 - 315, XP093118538, ISSN: 0022-328X, DOI: 10.1016/0022-328X(91)86477-8
- S. WEBER, ET AL.: "Reactions of triphenyltin hydride with thiophene compounds and certain carboxylic acids", JOURNAL OF ORGANIC CHEMISTRY, vol. 27, no. 4, 1 April 1962 (1962-04-01), American Chemical Society, Washington, DC, US, pages 1258 - 1260, XP093119288, ISSN: 0022-3263, DOI: 10.1021/jo01051a034
- T. OGAWA, ET AL.: "A stereocontrolled approach to the synthesis of glycosyl esters. Partial synthesis of stevioside from steviobioside", CARBOHYDRATE RESEARCH, vol. 60, no. 1, January 1978 (1978-01-01), Elsevier Scientific, Amsterdam, NL, pages C7 - C10, XP026618472, ISSN: 0008-6215, DOI: 10.1016/S0008-6215(00)83484-9
- WUTHIER URS, ET AL.: "Anion Selectivities of Triorganyltin Acetates and Halides in Solvent Polymeric Membranes", HELVETICA CHIMICA ACTA, vol. 69, no. 6, 10 September 1986 (1986-09-10), pages 1435 - 1441, XP055948449, DOI: 10.1002/hlca.19860690616
- ZIMMER HANS, ET AL.: "Sterically hindered Group IVa organometallics. VI. Preparation and Properties of Neophyltins and Related Compounds", THE JOURNAL OF ORGANIC CHEMISTRY, AMERICAN CHEMICAL SOCIETY, vol. 31, no. 11, 1 November 1966 (1966-11-01), pages 3857 - 3860, XP055948453, ISSN: 0022-3263, DOI: 10.1021/jo01349a515
- SASIN GEORGE S: "REACTIONS OF BIS-(TRIETHYLTIN) OXIDE WITH ACIDS. TRIETHYLTIN MERCAPTIDES", THE JOURNAL OF ORGANIC CHEMISTRY, AMERICAN CHEMICAL SOCIETY, vol. 18, no. 9, 1 September 1953 (1953-09-01), pages 1142 - 1145, XP055948454, ISSN: 0022-3263, DOI: 10.1021/jo50015a012
- CHIH HWA, PENFOLD B R: "Crystal and molecular structures of two polymeric organotin carboxylates, CsHI~O2Sn and CsHgF302Sn", JOURNAL OF CRYSTAL AND MOLECULAR STRUCTURE, SPRINGER,, US, vol. 3, 30 September 1973 (1973-09-30), US , pages 285 - 297, XP055948464, ISSN: 0308-4086
- G. A. RAZUVAEV ET AL.: "[Reaction of acyl peroxides with organic derivatives of lead, tin and silicon]", DOKLADY AKADEMII NAUK SSSR, vol. 137, no. 3, 31 December 1961 (1961-12-31), RU , pages 618 - 621, XP009538017, ISSN: 0002-3264
- VAN DER KERK G.J.M., J.G.A. LUIJTEN: "Investigations on organotin compounds. V The preparation and antifungal properties of unsymmetrical tri-n-alkyltin acetates", JOURNAL OF APPLIED CHEMISTRY, WILEY, US, vol. 6, no. 2, 1 February 1956 (1956-02-01), US , pages 56 - 60, XP055948467, ISSN: 0021-8871, DOI: 10.1002/jctb.5010060202
- RUDOLPH WILLEM ET AL.: "Synthesis, characterization and in vitro antitumor activity of triphenyl and tri-n-butyltin benzoates, phenylacetates and cinnamates", JOURNAL OF ORGANOMETALLIC CHEMISTRY, vol. 531, no. 1-2, 15 March 1997 (1997-03-15), XP004075035, ISSN: 0022-328X, DOI: 10.1016/S0022-328X(96)06686-7
- TOMOYA OGAWA ET AL.: "A stereocontrolled approach to the synthesis of glycosyl esters. Partial synthesis of stevioside from steviobioside", CARBOHYDRATE RESEARCH, vol. 60, no. 1, 31 January 1978 (1978-01-31), XP026618472, ISSN: 0008-6215, DOI: 10.1016/S0008-6215(00)83484-9
- XIE QINGLAN, ET AL.: "The Structure and Biological Activity of Tributyltin Carboxylates", ACTA CHIMICA SINICA, ZHONGGUO KEXUEYUAN SHANGHAI YOUJI HUAXUE YANJIUSUO, CN, vol. 49, no. 7, 30 July 1991 (1991-07-30), CN , pages 723 - 728, XP055948470, ISSN: 0567-7351

## Description

### TECHNICAL FIELD

The present disclosure belongs to the technical field of chemical manufacturing, and specifically relates to the use of a mono-tin organic compound.

### BACKGROUND

Sucralose, first synthesized by the British Tate&Lyte Group, has sweetness 600 times than that of sucrose. The sucralose has advantages of no calories, high sweetness, pure sweet taste, and high safety, and is also one of the most competitive sweeteners artificially synthesized so far.

Sucrose-6-carboxylate is a key intermediate in the synthesis of sucralose by monoester method. Currently, there have been mainly three kinds of production techniques for the sucrose-6-carboxylate. The first one is an orthoacetate method: acetylation occurs at 4- or 6-position, and then the acetyl at the 4-position is converted to an acetyl at the 6-position, generating sucrose- 6-acetate. The second one is an organotin monoester method: sucrose is reacted with an organotin compound as a catalyst to generate sucrose organotin ester, and then the sucrose organotin ester is reacted with an acylating agent to generate the sucrose-6-carboxylate. In this case, the catalyst organotin compound can be recycled. The third one is an enzymatic catalysis method: by using a catalytic performance of active enzymes, acetylation selectively occurs at the 6-position to generate the sucrose-6-carboxylate. In comparison, due to simple process, mild conditions, high yield, recyclable catalyst, and less exhaust gas, wastewater, and solid waste, the organotin monoester method has currently become a mainstream production process.

At present, in the organotin monoester method, organotin compounds that can be used as catalysts mainly include: di(hydrocarbyl)tin oxide, 1,3-bis(hydrocarbyloxy)-1,1,3,3-tetra-(hydrocarbyl)distannoxane, 1,3-diacyloxy-1,1,3,3-tetra-(hydrocarbyl)distannoxane, 1-acyloxy-1,1,3,3-tetra-(hydrocarbyl)distannoxane, and dihydrocarbyl tin diacyloxy. These organotin compounds each are double-tin compounds or multi-tin compounds. In the production, it is found that the di(hydrocarbyl)tin oxide can be reacted with acylating agents in different proportions, such as anhydrides, to gradually generate the 1-acyloxy-1,1,3,3-tetra-(hydrocarbyl)distannoxane, the 1,3-diacyloxy-1,1,3,3-tetra-(hydrocarbyl)distannoxane, and the dihydrocarbyl tin diacyloxy. These organotins in different acylation states exist as a mixture, and are difficult to be separated and purified effectively. Therefore, it is generally necessary to adjust a feed ratio of the organotin due to fine-tuning of the production process, and a non-strict process may lead to production fluctuations and seriously affect production stability.

In addition, due to the structure of ditin, organotin compounds are distributed in a light phase during extraction and recovery, which are difficult to be recovered completely, resulting in a loss of 1% to 5% of the catalyst each time during the reaction due to material entrainment and other reasons. As a result, new organotin compounds must be replenished for each production, and the entrained catalyst may have a bad influence on the subsequent chlorination reactions.

D2 ("The preparation and tin-119m Mossbauer spectra of tri-n-butylstannyl esters of heterocyclic and substituted aromatic carboxylic acids", JOURNAL OF ORGANOMETALLIC CHEMISTRY, Vol. 156, No. 2, 1978, pages 359-368) discloses preparation and tin-119m mössbauer spectra of tri-n-butylstannyl esters of heterocyclic and substituted aromatic carboxylic acids. Specifically, D2 discloses Bu₃SnOCOR, wherein R is phenyl.

D3 ("The coupling 2J(119Sn-O-13CO) in tributyltin acetate", JOURNAL OF ORGANOMETALLIC CHEMISTRY, Vol. 403, No. 3, 1991, pages 311-315) discloses the preparation of Bu₃SnOCOCH₃.

Organotin with a tributyltin structure can also be used as an esterification catalyst, which is easier to be recycled and applied. In addition, due to the structure of ditin, organotin compounds are distributed in a light phase during extraction and recovery, which are difficult to be recovered completely, resulting in a loss of 1% to 5% of the catalyst each time during the reaction due to material entrainment and other reasons. As a result, new organotin compounds must be replenished for each production, and the entrained catalyst may have a bad influence on the subsequent chlorination reactions. Moreover, the structure of tritin, such as common bis(tri-n-butyltin) oxide, bis(tri-n-butyltin) oxide, and hexabutyl distannoxane, also has dimerization.

### SUMMARY

In view of the above problems, a mono-tin organic compound and a preparation method and use thereof are provided in the present disclosure to overcome the above problems or at least partially solve the above problems.

The present disclosure provides use of a mono-tin organic compound in the synthesis of sucrose-6-carboxylate, the compound having a structure shown in formula (1): 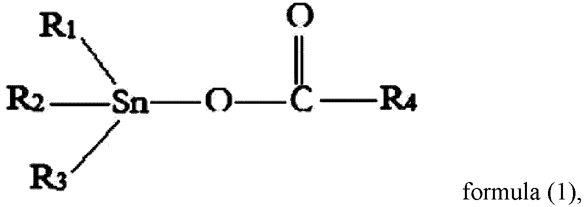 wherein R₁, R₂, and R₃ each are independently selected from the group consisting of C₁ to C₈ linear or branched saturated alkyl, C₂ to C₈ linear or branched unsaturated alkyl, C₃ to C₈ substituted or unsubstituted saturated cycloalkyl, C₃ to C₈ substituted or unsubstituted unsaturated cycloalkyl, and C₆ to C₁₂ aryl or substituted aryl; and R₄ is selected from the group consisting of C₁ to C₆ linear or branched saturated alkyl and C₆ to C₁₂ aryl or substituted aryl; and wherein the sucrose-6-carboxylate is synthesized by a method including: using the mono-tin organic compound as a catalyst and conducting dehydration esterification on sucrose in a polar aprotic solvent to obtain a sucrose organotin ester-containing solution.

In summary, the present disclosure has beneficial effects as follows: a mono-tin organic compound is provided, which can be used for the synthesis of sucrose-6-carboxylate by an organotin method. During the synthesis of the sucrose-6-carboxylate, the use of mono-tin organic compound enables accurate dosing, and results in improved catalyst recovery rate, reduced occurrence of subsequent chlorination side reactions, and moreover rapid reaction, a low energy consumption, and a higher yield per unit volume catalyst. Also, the use of mono-tin organic compound results in saved floor space, simplified process, and reduced waste discharge and treatment, which is environmentally-friendly.

The above description is merely a summary of the technical solution of the present disclosure. In order to make the technical means of the present disclosure to be understood more clearly and implementable in accordance with the content of the specification, and in order to make the above and other objectives, features and advantages of the present disclosure more obvious and easier to understand, specific embodiments of the present disclosure are described below.

### DETAILED DESCRIPTION OF THE EMBODIMENTS

Exemplary embodiments of the present disclosure will be described in detail below with reference to the accompanying drawings. Although the accompanying drawings show exemplary embodiments of the present disclosure, it should be understood that the present disclosure may be implemented in various forms and should not be limited to the embodiments set forth herein. Instead, these embodiments are provided for a thorough understanding of the present disclosure, and a scope of the present disclosure can be fully conveyed to those skilled in the art.

An idea of the present disclosure is that: in the prior art, organotin compounds, as a catalyst for synthesizing sucrose-6 carboxylate, have different acylation and polymerization states, which may lead to varied compounding and then adversely affect the production, thus resulting in unsatisfactory yield of sucrose-6 carboxylate, complex products, waste of catalysts, and frequent side reactions in subsequent processes. In view of this, the present disclosure provides a mono-tin organic compound. The mono-tin organic compound can be used as a catalyst for the synthesis of sucrose-6 carboxylate to effectively overcome the above disadvantages.

In the present disclosure, a mono-tin organic compound is provided for the synthesis of sucrose-6-carboxylate, wherein the compound has a structure shown in formula (1): 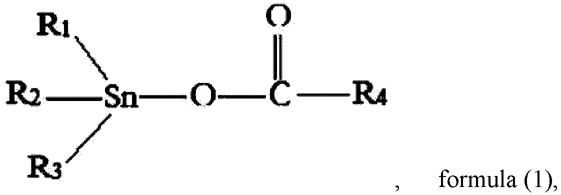 wherein R₁, R₂, and R₃ each are independently selected from the group consisting of C₁ to C₈ linear or branched saturated alkyl, C₂ to C₈ linear or branched unsaturated alkyl, C₃ to C₈ substituted or unsubstituted saturated cycloalkyl, C₃ to C₈ substituted or unsubstituted unsaturated cycloalkyl, and C₆ to C₁₂ aryl or substituted aryl; and R₄ is selected from the group consisting of C₁ to C₆ linear or branched saturated alkyl and C₆ to C₁₂ aryl or substituted aryl. In some other embodiments, R₁, R₂, and R₃ each represent C₁ to C₆ linear or branched saturated alkyl. In some other embodiments, R₁, R₂, and R₃ each represent n-butyl. In some other embodiments R₄ represents methyl and phenyl.

More specifically, in some embodiments of the present disclosure, the C₁ to C₈ linear or branched saturated alkyl is methyl, ethyl, propyl, isopropyl, butyl, isobutyl, tert-butyl, pentyl, isopentyl, neopentyl, n-hexyl, branched hexyl, n-heptane, branched heptane, n-octane, and branched octane.

In some embodiments of the present disclosure, the C₂ to C₈ linear or branched unsaturated alkyl is vinyl, propenyl, butenyl, isobutenyl, tert-butenyl, pentenyl, isopentenyl, neopentenyl, n-hexenyl, branched hexenyl, n-heptenyl, branched heptenyl, n-octenyl, branched octenyl, ethynyl, propynyl, butynyl, isobutynyl, tert-butynyl, pentynyl, isopentynyl, neopentynyl, n-hexynyl, branched hexynyl, n-heptynyl, branched heptynyl, n-octynyl, and branched octynyl.

In some embodiments of the present disclosure, the C₃ to C₈ substituted or unsubstituted saturated cycloalkyl is cyclopropyl, cyclobutyl, cyclopentyl, cyclohexyl; monosubstituted cyclopropyl, such as methylcyclopropyl and ethylcyclopropyl; disubstituted cyclopropyl, such as dimethylcyclopropyl and methylethylcyclopropyl; monosubstituted cyclobutyl, disubstituted cyclobutyl, and trisubstituted cyclobutyl, such as trimethylcyclobutyl; monosubstituted cyclopentyl, disubstituted cyclopentyl, and trisubstituted cyclopentyl, such as methylethylcyclopentyl; and monosubstituted cyclohexyl and disubstituted cyclohexyl, such as methylcyclohexyl.

In some embodiments of the present disclosure, the C₃ to C₈ substituted or unsubstituted unsaturated cycloalkyl is cyclopropenyl, cyclobutenyl, cyclopentenyl, cyclohexenyl, cyclopropynyl, cyclobutynyl, cyclopentynyl, cyclohexynyl, monosubstituted cyclopropenyl, and disubstituted cyclopropenyl, such as methylcyclopropenyl; monosubstituted cyclobutenyl, disubstituted cyclobutenyl, and trisubstituted cyclobutenyl, such as dimethylcyclobutenyl; monosubstituted cyclopentenyl, disubstituted cyclopentenyl, and trisubstituted cyclopentenyl, such as methylethylcyclopentenyl; monosubstituted cyclohexenyl and disubstituted cyclohexenyl, such as methylcyclohexenyl; monosubstituted cyclopropynyl and disubstituted cyclopropynyl, such as dimethylcyclopropynyl; monosubstituted cyclobutynyl, disubstituted cyclobutynyl, and trisubstituted cyclobutynyl, such as ethylcyclobutynyl; monosubstituted cyclopentynyl, disubstituted cyclopentynyl, and trisubstituted cyclopentynyl, such as methylethylcyclopentynyl; and monosubstituted cyclohexynyl and disubstituted cyclohexynyl, such as methylcyclohexynyl.

In some embodiments of the present disclosure, the C₆ to C₁₂ aryl or substituted aryl is phenyl, monosubstituted phenyl, disubstituted phenyl, trisubstituted phenyl, tetrasubstituted phenyl, pentasubstituted phenyl, and hexasubstituted phenyl, such as methylethylphenyl, benzocyclobutyl, and benzocyclopentyl.

In some embodiments of the present disclosure, R₄ represents C₁ to C₆ linear or branched saturated alkyl, and is selected from the group consisting of methyl, ethyl, propyl, isopropyl, butyl, isobutyl, tert-butyl, pentyl, isopentyl, neopentyl, n-hexyl, and branched hexyl.

In some embodiments of the present disclosure, R₄ represents C₆ to C₁₂ aryl or substituted aryl, and is selected from the group consisting of phenyl, monosubstituted phenyl, disubstituted phenyl, trisubstituted phenyl, tetrasubstituted phenyl, pentasubstituted phenyl, and hexasubstituted phenyl, such as methylethylphenyl, benzocyclobutyl, and benzocyclopentyl.

In the present disclosure, the mono-tin organic compound can be used for the synthesis of sucrose-6-carboxylate by an organotin method, and during the synthesis of the sucrose-6-carboxylate, the use of mono-tin organic compound enables accurate dosing, and results in improved catalyst recovery rate, and reduced occurrence of subsequent chlorination side reactions.

The present disclosure further provides a method for preparing any one of the above mono-tin organic compounds, where the mono-tin organic compound can be used as a catalyst for synthesizing sucrose-6-carboxylate by an organotin method. In the method, a compound represented by formula (2) is used as a raw material, which has only one active site that can be acylated, so it does not need to consider the situation of organotins with different acylation states. This method can achieve relatively-pure acetoxy-tributyltin, and has advantages of stable feeding ratio, strict process, no fluctuation in production, and stable production; moreover, it has simple process, readily-available raw materials, and high yield. The method includes the following steps:
dissolution: dissolving trialkyltin oxide in an organic solvent to obtain an organic reaction solution; where the trialkyltin oxide is a compound shown in formula (2): 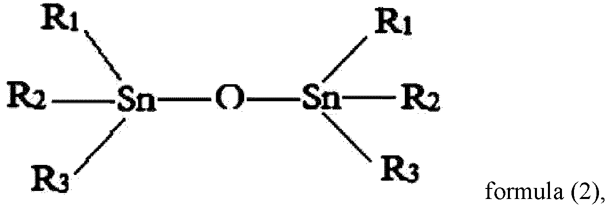
wherein R₁, R₂, and R₃ each are independently selected from the group consisting of C₁ to C₈ linear or branched saturated alkyl, C₂ to C₈ linear or branched unsaturated alkyl, C₃ to C₈ substituted or unsubstituted saturated cycloalkyl, C₃ to C₈ substituted or unsubstituted unsaturated cycloalkyl, and C₆ to C₁₂ aryl or substituted aryl, preferably n-butyl; and
acylation: under preset conditions, adding an acylating agent to the organic reaction solution and holding for a preset time, such that the trialkyltin oxide and the acylating agent are subjected to acylation; where the acylating agent is selected from the group consisting of a compound shown in formula (3) and a corresponding anhydride thereof:

   R₄-COOH, formula (3),
wherein R₄ is selected from the group consisting of C₁ to C₆ linear or branched saturated alkyl and C₆ to C₁₂ aryl or substituted aryl, preferably methyl and phenyl.

In some embodiments, R₁, R₂, and R₃ each represent C₁ to C₆ linear or branched saturated alkyl, preferably n-butyl.

More specifically, in some embodiments of the present disclosure, the C₁ to C₈ linear or branched saturated alkyl is methyl, ethyl, propyl, isopropyl, butyl, isobutyl, tert-butyl, pentyl, isopentyl, neopentyl, n-hexyl, branched hexyl, n-heptane, branched heptane, n-octane, and branched octane.

In some embodiments of the present disclosure, the C₂ to C₈ linear or branched unsaturated alkyl is vinyl, propenyl, butenyl, isobutenyl, tert-butenyl, pentenyl, isopentenyl, neopentenyl, n-hexenyl, branched hexenyl, n-heptenyl, branched heptenyl, n-octenyl, branched octenyl, ethynyl, propynyl, butynyl, isobutynyl, tert-butynyl, pentynyl, isopentynyl, neopentynyl, n-hexynyl, branched hexynyl, n-heptynyl, branched heptynyl, n-octynyl, and branched octynyl.

In some embodiments of the present disclosure, C₃ to C₈ substituted or unsubstituted saturated cycloalkyl is cyclopropyl, cyclobutyl, cyclopentyl, cyclohexyl; monosubstituted cyclopropyl, such as methylcyclopropyl and ethylcyclopropyl; disubstituted cyclopropyl, such as dimethylcyclopropyl and methylethylcyclopropyl; monosubstituted cyclobutyl, disubstituted cyclobutyl, and trisubstituted cyclobutyl, such as trimethylcyclobutyl; monosubstituted cyclopentyl, disubstituted cyclopentyl, and trisubstituted cyclopentyl, such as methylethylcyclopentyl; and monosubstituted cyclohexyl and disubstituted cyclohexyl, such as methylcyclohexyl.

In some embodiments of the present disclosure, the C₃ to C₈ substituted or unsubstituted unsaturated cycloalkyl is cyclopropenyl, cyclobutenyl, cyclopentenyl, cyclohexenyl, cyclopropynyl, cyclobutynyl, cyclopentynyl, cyclohexynyl, monosubstituted cyclopropenyl, and disubstituted cyclopropenyl, such as methylcyclopropenyl; monosubstituted cyclobutenyl, disubstituted cyclobutenyl, and trisubstituted cyclobutenyl, such as dimethylcyclobutenyl; monosubstituted cyclopentenyl, disubstituted cyclopentenyl, and trisubstituted cyclopentenyl, such as methylethylcyclopentenyl; monosubstituted cyclohexenyl and disubstituted cyclohexenyl, such as methylcyclohexenyl; monosubstituted cyclopropynyl and disubstituted cyclopropynyl, such as dimethylcyclopropynyl; monosubstituted cyclobutynyl, disubstituted cyclobutynyl, and trisubstituted cyclobutynyl, such as ethylcyclobutynyl; monosubstituted cyclopentynyl, disubstituted cyclopentynyl, and trisubstituted cyclopentynyl, such as methylethylcyclopentynyl; and monosubstituted cyclohexynyl and disubstituted cyclohexynyl, such as methylcyclohexynyl.

In some embodiments of the present disclosure, the C₆ to C₁₂ aryl or substituted aryl is phenyl, monosubstituted phenyl, disubstituted phenyl, trisubstituted phenyl, tetrasubstituted phenyl, pentasubstituted phenyl, and hexasubstituted phenyl, such as methylethylphenyl, benzocyclobutyl, and benzocyclopentyl.

The method further includes acylation: under preset conditions, adding an acylating agent to the organic reaction solution and holding for a preset time, such that the compound shown in formula (2) and the acylating agent are subjected to acylation, wherein the acylating agent is a compound shown in formula (3) and a corresponding anhydride thereof:

R₄-COOH formula (3),

wherein the R₄ is selected from the group consisting of C₁ to C₆ linear or branched saturated alkyl and C₆ to C₁₂ aryl or substituted aryl. In some embodiments of the present disclosure, R₄ is selected from the group consisting of methyl, ethyl, propyl, isopropyl, butyl, isobutyl, tert-butyl, pentyl, isopentyl, neopentyl, n-hexyl, and branched hexyl, preferably methyl and phenyl.

In the present disclosure, ditin organic oxides are converted into mono-tin organic oxides through acylation, thereby avoiding the instability of batching and incomplete recovery caused by different acylation and polymerization states of organic ditin catalysts in the prior art, which adversely affects production, cause waste and side reactions, so as to overcome the drawbacks of unsatisfactory yield, more side reactions, catalyst waste, and side reactions in the subsequent reaction.

In some embodiments of the present disclosure, the method for preparing the mono-tin organic compound further includes: post-treatment: subjecting a reaction solution obtained from the acylation to a reduced-pressure distillation until no fraction is distilled out, and recovering the organic solvent and an unreacted acylating agent to obtain the mono-tin organic compound; wherein the reduced-pressure distillation is conducted at a temperature of 40 °C to 120 °C, preferably 60 °C to 100 °C and a pressure of -0.050 MPa to -0.099 MPa, preferably -0.080 MPa to -0.095 MPa.

After the acylation is finished, the organic solvent and the unreacted acylating agent can be recovered by means such as distillation, so as to obtain a target product with higher purity, namely the mono-tin organic compound. In some embodiments of the present disclosure, there is no limitation on distillation conditions. In consideration of distillation efficiency and energy consumption, in other embodiments, the reduced-pressure distillation can be conducted at a temperature of 40 °C to 120 °C and a pressure of -0.050 MPa to -0.099 MPa. In other embodiments, the reduced-pressure distillation can be conducted at a temperature of 60 °C to 100 °C and a pressure of -0.080 MPa to -0.095 MPa. If the distillation temperature is less than 40 °C and the distillation pressure is greater than -0.050 MPa, the temperature is too low and the distillation pressure is too high, so that the solvent distillation rate is too low, and the degree of distillation is not thorough enough, resulting in a poor separation effect. If the distillation temperature is greater than 120 °C and the distillation pressure is less than -0.099 MPa, the solvent distillation rate is too large, so that the distillation process is difficult to control, and the target product is easily distilled out together with the organic solvent, resulting in a decrease in yield.

### Type and amount of the organic solvent

In some embodiments of the present disclosure, during the dissolving, there is no limitation on the type of the organic solvent, as long as the acylation could be realized. In some embodiments, the organic solvent is one or more selected from the group consisting of cyclohexane, hexane, acetonitrile, ethyl acetate, toluene, trichloroethane, dichloromethane, dichloroethane, chloroform, DMF, and DMAC. In other embodiments, the organic solvent is cyclohexane.

In some embodiments of the present disclosure, there is no limitation on the amount of organic solvent. In other embodiments, a ratio of the volume of the organic solvent to the mass of the trialkyltin oxide is in a range of (0.2-6.0): 1. In still other embodiments, a ratio of the volume of the organic solvent to the mass of the trialkyltin oxide is in a range of (2.0-3.0): 1. The amount of organic solvent can be set with a bottom line that can enable a complete acylation. If the volume of the organic solvent is less than 0.2 times the mass of the trialkyltin oxide, the amount is too small to ensure smooth progress of the acylation. If the volume of the organic solvent is more than 6.0 times the mass of the trialkyltin oxide, the amount is too large, causing unnecessary waste, and making the whole process have a large amount of disposal, the follow-up treatment process is complicated, and the distillation is difficult, which cannot bring other beneficial effects.

### Amount of the acylating agent

In some embodiments of the present disclosure, only one active site of the trialkyltin oxide can undergo acylation, without needs to consider situations of organotin in different acylation states. Therefore, in the acylation, the amount of the acylating agent is not limited. In some embodiments, the acylating agent is used in a slightly excessive amount to increase a conversion rate of trialkyltin oxide. In other embodiments, a mass ratio of the acylating agent to the trialkyltin oxide is in a range of (0.1-1): 1. In yet other embodiments, the mass ratio is in the range of (0.4-0.6): 1. If the mass of the acylating agent is less than 10% that of the trialkyltin oxide, the amount of the acylating agent is too low, resulting in too low conversion rate of the trialkyltin oxide. If the mass of the acylating agent is greater than 100% that of the trialkyltin oxide, the amount of the acylating agent is too large, which does not further improve the conversion rate of the trialkyltin oxide but causes unnecessary waste.

### Conditions for the acylation

In some embodiments of the present disclosure, there is no limitation on conditions of the acylation. In some other embodiments, under stirring, the acylation is conducted at a preset temperature of 0 °C to 80 °C for a preset time of 1 min to 24 h. In some other embodiments, the acylation is conducted at a preset temperature of 30 °C to 50 °C for a preset time of 30 min to 2 h. If the acylation is conducted at lower than 0°C for shorter than 1 min, the acylation conditions are too passivated and the time is too short, making it difficult to conduct the acylation, and the conversion rate of the reactant is too low, resulting in waste of the reactant. If the acylation is conducted at a temperature higher than 80 °C for a time longer than 2 h, the acylation temperature may be too high and the time may be too long, making the acylation conditions too intense, which is prone to side reactions and even causes dehydration and coke formation reactions.

According to some embodiments of the present disclosure, use of the mono-tin organic compound in the synthesis of sucrose-6-carboxylate is further provided. Any one of the above mono-tin organic compounds is used as a catalyst to synthesize sucrose-6-carboxylate. The use includes conducting dehydration esterification on the mono-tin organic compound and sucrose in a polar aprotic solvent, to obtain a sucrose organotin ester-containing solution. The mono-tin organic compound is used to synthesize sucrose-6-carboxylate, thereby avoiding the instability of batching and incomplete recovery caused by different acylation and polymerization states of organic ditin catalysts in the prior art, which adversely affects production, causes waste and side reactions, so as to overcome the drawbacks such as unsatisfactory yield, more side reactions, catalyst waste, and side reactions in the subsequent reaction.

Due to the structural characteristics of mono-tin organic compound, during extraction and recovery, less mono-tin organic compound is distributed in the light phase, which is easy to achieve complete recovery, resulting in a loss of only less than about 2% of the catalyst each time during the reaction, which results from situations such as material entrainment. If the extraction is well controlled, the loss of the catalyst can be controlled to be below 1% each time. Therefore, there is almost no need to replenish new catalysts for each production, and the generated sucrose-6-carboxylate has a high purity, and shows a better effect in the subsequent chlorination for preparing sucralose.

### Dehydration esterification process

In some embodiments of the present disclosure, the dehydration esterification is conducted by any one of solvent azeotropy, negative-pressure distillation, atomization drying dehydration, and vacuum tower dehydration.

The atomization drying dehydration can be briefly described as follows: including preparation of a reaction solution: adding sucrose and a catalyst to a polar aprotic solvent, heating a resulting mixture for dissolution, and adding a non-polar solution to obtain a reaction mixed solution; atomization: subjecting the reaction solution to atomization to obtain droplets; dehydration: thoroughly mixing the droplets with a gasified dehydration medium such that the droplets undergo a dehydration reaction to obtain an intermediate mixture of sucrose organotin ester-containing droplets; and separation: subjecting the intermediate mixture to a separation to obtain a sucrose organotin ester solution and a dehydrated gas-liquid mixture. The method further includes cycling: recovering the sucrose organotin ester solution obtained from the separation step and cycling the sucrose organotin ester solution to the atomization and dehydration steps several times.

In the vacuum tower dehydration, the vacuum tower can be a packed tower or a bubble cap tower. The dehydration includes: preparation of a mixed solution: dissolving sucrose and an organotin compound in a solvent by heating to obtain a reaction mixed solution; dehydration: feeding the reaction mixed solution into a vacuum tower reactor through a liquid inlet of the vacuum tower reactor, contacting and reacting with a dehydration medium which is fed through a gas inlet of the vacuum tower reactor, subjecting the reaction mixed solution to a dehydration reaction and a gas-liquid exchange with the dehydration medium, thereby obtaining the sucrose organotin ester solution and a dehydration medium gas containing water vapor; wherein the sucrose organotin ester solution is discharged from the liquid outlet of the vacuum tower reactor; and the dehydration medium gas containing water vapor is discharged from the gas outlet of the vacuum tower reactor.

In the dehydration by solvent azeotropy, since a part of the non-polar organic solvent can form an azeotrope with water, the water produced by the reaction is taken out of the reaction system by forming an azeotrope during the distillation of the non-polar solvent, thereby achieving the dehydration. The process includes: preparation of a reaction solution: dissolving sucrose and a catalyst in a polar aprotic solvent by heating, and adding a non-polar solvent therein to obtain a reaction solution; dehydration: heating and distilling the reaction solution, where water produced by the reaction is taken out of the reaction system by forming an azeotrope during the distillation of the non-polar solvent to obtain a sucrose organotin ester solution; separation: after layering, separating the distilled non-polar organic solvent from the water phase, where the non-polar organic solvent can be recycled after separating water out and removing water to a certain extent. Further, the fully reacted sucrose organotin ester solution is obtained through azeotropic distillation with the non-polar organic solvent, water separation, drying, and recycling.

In the dehydration by negative-pressure distillation, by using a relationship between the boiling point and pressure of polar organic solvents, distillation can be conducted at negative pressure and lower temperature, and water produced by the reaction is entrained out of the reaction system during the distillation of polar solvents, thereby achieving dehydration. The process includes: preparation of a reaction solution: dissolving sucrose and a catalyst in a polar aprotic solvent by heating to obtain a reaction solution; dehydration: heating the reaction solution to conduct negative-pressure distillation, where water produced by the reaction is entrained out of the reaction system during the negative-pressure distillation of the polar solvent, to obtain a sucrose organotin ester solution; separation: subjecting a distilled polar organic solvents containing water to a dehydration-separation by related technologies, which includes distillation or using appropriate desiccants, and the polar organic solvents can be recycled after dehydration to a certain extent. Further, the fully reacted sucrose organotin ester solution is obtained through negative-pressure distillation with polar organic solvents, water separation, drying, and recycling.

### Reaction conditions for the dehydration esterification

In some embodiments of the present disclosure, there is no limitation on reaction conditions of the dehydration esterification. In some other embodiments, the dehydration esterification is conducted at a temperature of 60 °C to 120 °C and a pressure of 0.01 kPa to 50 kPa for 0.1 min to 8 h. In still some embodiments, the dehydration esterification is conducted at a temperature of 70 °C to 100 °C and a pressure of 0.5 kPa to 20 kPa for 1 min to 1 h.

### Type and amount of the polar aprotic solvent

The polar aprotic solvent is mainly used to dissolve sucrose and provide a reaction environment for the dehydration esterification. In some embodiments of the present disclosure, there is no limitation on the type of the polar aprotic solvent. In other embodiments, the polar aprotic solvent is one or more selected from the group consisting of dimethylsulfoxide, N-methylpyrrolidone, N,N-dimethylacetamide, hexamethylphosphoramide and N,N-dimethylformamide. In still some embodiments, the polar aprotic solvent is N,N-dimethylformamide.

In some embodiments of the present disclosure, there is no limitation on the amount of the polar aprotic solvent. In some other embodiments, a ratio of the volume of the polar aprotic solvent to the mass of the sucrose is in the range of (2-20) mL: 1 g. In yet other embodiments, the ratio is (3-10) mL: 1 g. In yet other embodiments, the ratio is (4-8) mL: 1 g. If the volume of the polar aprotic solvent is less than 2 times the mass of the sucrose, the amount of the polar aprotic solvent is too low, the sucrose cannot be completely dissolved, resulting in a waste of sucrose raw material, which would adversely affect the heat transfer and mass transfer during subsequent reactions since the sucrose exists in a solid form. If the volume of the mixed solvent is more than 20 times the mass of the sucrose, the amount of the mixed solvent is too large, which results in a waste of raw materials, and brings great trouble to the subsequent solvent treatment and even causes excessive energy consumption, increased production costs, reduced efficiency, and environmental pollution.

### Amount of the catalyst

In the present disclosure, the mono-tin organic compound is used as a catalyst to synthesize sucrose-6-carboxylate. In some embodiments, there is no limitation on the amount of catalyst. In other embodiments, a molar ratio of the mono-tin organic compound to the sucrose is in a range of (0.3-3): 1. In yet other embodiments, the molar ratio is in the range of (0.5-2.0): 1. In yet other embodiments, the molar ratio is in the range of (0.9-1.2): 1. If the molar amount of the mono-tin organic compound catalyst is less than 30% that of the sucrose, the amount of the mono-tin organic compound catalyst is too low, resulting in a large amount of sucrose reactant unable to participate in the reaction, which is not conducive to the forward progress of the reaction. If the molar amount of mono-tin organic compound is more than 300% that of sucrose, the amount of mono-tin organic compound is too large, resulting in di-esterification or polyesterification.

According to some embodiments of the present disclosure, the use of the mono-tin organic compound as a catalyst in the synthesis of sucrose-6-carboxylate further includes: cooling the sucrose organotin ester-containing solution, and conducting acylation with the acylating agent to obtain the sucrose-6-carboxylate.

The sucrose organotin ester is an intermediate for the preparation of sucrose-6-carboxylate from sucrose, and the sucrose-6-carboxylate can be obtained after acylation with an acylating agent. The acylating agent can be any one selected from the group consisting of acetic anhydride, butyric anhydride, benzoic anhydride, stearic anhydride, and lauric anhydride. The sucrose organotin ester solution is cooled and then subjected to acylation with the acylating agent to obtain sucrose-6-carboxylate. According to the type of acylating agent, the corresponding sucrose-6-carboxylates obtained are sucrose-6-acetate, sucrose-6-butyrate, sucrose-6-benzoate, sucrose-6-fatty acid ester, and sucrose-6-laurate, respectively. The sucrose-6-acetate and sucrose-6-benzoate can be used as raw materials for synthesizing other sucrose-6-carboxylates and can also be used as intermediates for synthesizing a sweetener sucralose; the other types of sucrose-6-carboxylate can be used as food additives, chemical products, and synthetic intermediates for other reactions.

### Conditions for the acylation

In some embodiments of the present disclosure, there is no limitation on reaction conditions of the acylation. In some embodiments, the acylation is conducted at a temperature of 0 °C to 50 °C for 10 min to 24 h. In other embodiments, the acylation is conducted at a temperature of 5 °C to 20 °C for 30 min to 4 h. If the acylation is conducted at a temperature lower than 0° C for shorter than 30 min, the temperature is too low and the time is too short, resulting in incomplete acylation, waste of raw materials, and low product yield. If the acylation is conducted at a temperature higher than 50 °C for longer than 4 h, the temperature is too high and the time is too long, causing excessive acylation and even coke formation.

### Test method involved in the present disclosure

In each example and comparative example of the present disclosure, the content or purity of each substance is measured by high-performance liquid chromatography (HPLC) under the following conditions, and will not be repeated in each example.

Analysis and determination conditions of HPLC: High-performance liquid chromatography of Shimadzu, Japan: RID-10A differential refractive index detection, LC-10ADVP high-pressure pump, and CTO-10ASVP incubator; chromatographic column: Agilent XDB C18 column (250 mm × 4.6 mm, 5 µm); mobile phase: methanol-0.125% aqueous solution of dipotassium hydrogen phosphate (4:6); column temperature: 30 °C; and flow rate: 1.0 mL/min. Methanol (chromatographically pure), dipotassium hydrogen phosphate (analytically pure), and ultrapure water (UPW) are required, each of which is a reference material, and a content is determined by an external standard method.

### Example 1: preparation of catalyst 1A

61.5 g of a compound shown in formula (2) (wherein R₁, R₂, and R₃ each represent n-butyl) and 200 mL of cyclohexane were added into a 500 mL three-necked flask, and stirred and heated to 40 °C until clear.

40 mL of acetic anhydride was slowly added into the three-necked flask within about 30 min. A resulting mixed solution was heated to 60 °C, and maintained at this temperature for 3 h for dissolution.

A reacted solution was subjected to a reduced-pressure distillation at -0.095 MPa and 80 °C until no component was distilled out to complete the reduced-pressure distillation. 170 mL of the solvent was recycled, which contained a small amount of the acetic anhydride. A mono-tin organic compound, i.e., acetoxy-tributyltin, was obtained, designated as the catalyst 1A.

### Example 2: preparation of catalyst 1B

61.5 g of a compound shown in formula (2) (wherein R₁, R₂, and R₃ each represent n-butyl) and 200 mL of cyclohexane were added into a 500 mL three-necked flask, and stirred and heated to 40 °C until clear.

100 mL of benzoic anhydride was slowly added into the three-necked flask within about 30 min. A resulting mixed solution was heated to 60 °C, and maintained at this temperature for 3 h for dissolution.

A reacted solution was subjected to a reduced-pressure distillation at -0.099 MPa and 100 °C until no component was distilled out to complete the reduced-pressure distillation. 175 mL of the solvent was recovered, which contained a small amount of the benzoic anhydride. A mono-tin organic compound, i.e., benzoyloxy-tributyltin, was obtained, designated as the catalyst 1B.

### Example 3: preparation of catalyst 1C

80.0 g of a compound shown in formula (2) (wherein R₁, R₂, and R₃ each represent phenyl) and 200 mL of toluene were added into a 500 mL three-necked flask, and stirred and heated to 40 °C until clear.

40 mL of acetic anhydride was slowly added into the three-necked flask within about 30 min. A resulting mixed solution was heated to 60 °C, and maintained at this temperature for 3 h for dissolution.

A reacted solution was subjected to a reduced-pressure distillation at -0.095 MPa and 80 °C until no component was distilled out to complete the reduced-pressure distillation. 170 mL of the solvent was recovered, which contained a small amount of the acetic anhydride. A mono-tin organic compound, i.e., acetoxy-triphenyltin, was obtained, designated as the catalyst 1C.

### Example 4: preparation of catalyst 1D

80.0 g of a compound shown in formula (2) (wherein R₁, R₂, and R₃ each represent phenyl) and 200 mL of toluene were added into a 500 mL three-necked flask, and stirred and heated to 40 °C until clear.

100 mL of benzoic anhydride was slowly added into the three-necked flask within about 30 min. A resulting mixed solution was heated to 60 °C, and maintained at this temperature for 3 h for dissolution.

A reacted solution was subjected to a reduced-pressure distillation at -0.099 MPa and 80 °C until no component was distilled out to complete the reduced-pressure distillation. 170 mL of the solvent was recovered, which contained a small amount of the benzoic anhydride. A mono-tin organic compound, i.e., benzoyloxy-triphenyltin, was obtained, designated as the catalyst 1D.

### Example 5: use of catalyst 1A in the preparation of sucrose-6-carboxylate

The mono-tin organic compound acetoxy-tributyltin prepared in Example 1 was used as a catalyst, that is, catalyst 1A. 70 g of sucrose and 300 mL of DMF were added to the catalyst, and then the resulting mixture was heated to 80 °C and dissolved to obtain a solution. Negative-pressure dehydration was conducted at -95 KPa and 70 °C, while anhydrous DMF was continuously supplemented until the distilled DMF solvent had a water content of below 100 ppm, and the negative-pressure dehydration was stopped. This process required about 3 h.

A sucrose content of a resulting solution reaction system was calculated to be about 20 wt%. The acetic anhydride was added dropwise at a temperature lower than 10 °C with a ratio of the mass of sucrose to the molar of acetic anhydride being 1.0:1.1 to conduct acylation. After acylation at a temperature lower than 10 °C for 2 h, water was added with a ratio of the volume of the water to the total volume of a reaction solution being 0.25:1 to quench the reaction. Cyclohexane was added with a ratio of the volume of cyclohexane to the total volume of an obtained solution reaction system being 1:1, so as to extract the acetoxy-tributyltin, and the obtained sucrose-6-acetate solution was analyzed by HPLC. The normalization below and in the following examples meant that, when a mixture was subjected to separation assay by HPLC, a sum of all substances was specified as 100%, and a percentage of each substance to all substances was determined according to a peak area. The product distribution was shown in Table 1.

### Example 6: use of catalyst 1B in the preparation of sucrose-6-carboxylate

The mono-tin organic compound benzoyloxy-tributyltin prepared in Example 2 was used as a catalyst, that is, catalyst 1B. 70 g of sucrose, 300 mL of DMF, and 200 mL of cyclohexane were added to the catalyst, and then heated to 80 °C and dissolved to obtain a solution. Solvent azeotropy-based dehydration was conducted at -50 KPa and 65 °C, while anhydrous cyclohexane was continuously supplemented until the distilled cyclohexane had no obvious water phase (with a water content below 100 ppm), and the solvent azeotropy-based dehydration was stopped. This process required about 2 h.

A sucrose content of a resulting solution reaction system was calculated to be about 20 wt%. The benzoic anhydride was added dropwise at a temperature lower than 10 °C with a ratio of the mass of the sucrose to the molar mass of benzoic anhydride being 1.0:1.05 to conduct acylation. After acylation at a temperature lower than 10 °C for 2 h, water was added with a ratio of the volume of the water to the total volume of a reaction solution being 0.25:1 to quench the reaction. Cyclohexane was added with a ratio of the volume of cyclohexane to the total volume of an obtained solution reaction system being 1:1, so as to extract the benzoyloxy-tributyltin, and an obtained sucrose-6-benzoate solution was analyzed by HPLC. The product distribution was shown in Table 1.

### Example 7: use of catalyst 1C in the preparation of sucrose-6-carboxylate

The mono-tin organic compound acetoxy-triphenyltin prepared in Example 3 was used as a catalyst, that is, catalyst 1C. 70 g of sucrose and 300 mL of DMAC were added to the catalyst, and then heated to 80 °C and dissolved to obtain a solution. Spray negative-pressure dehydration was conducted at -95 KPa and 120 °C, and 4 levels of spray negative-pressure dehydration was conducted. This process required about 1 h.

A sucrose content of a resulting solution reaction system was calculated to be about 20 wt%. The hexane was added with a ratio of the volume of hexane to the total volume of the solution reaction system being 1:2. The acetic anhydride was added dropwise at a temperature lower than 10 °C with a ratio of the mass of sucrose to the mass of acetic anhydride being 1.0:1.10 to conduct acylation. After acylation at a temperature lower than 10 °C for 2 h, water was added with a ratio of the volume of the water to the total volume of a reaction solution being 0.25:1 to quench the reaction. Cyclohexane was added with a ratio of the volume of cyclohexane to the total volume of an obtained solution reaction system being 1:1, so as to extract the acetoxy-triphenyltin, and an obtained sucrose-6-acetate solution was analyzed by HPLC. The product distribution was shown in Table 1.

### Example 8: use of catalyst 1D in the preparation of sucrose-6-carboxylate

The mono-tin organic compound benzoyloxy-triphenyltin prepared in Example 4 was used as a catalyst, that is, catalyst 1D. 70 g of sucrose, 300 mL of DMF, and 200 mL of cyclohexane were added to the catalyst, and then heated to 80 °C and dissolved to obtain a solution. Solvent azeotropy-based dehydration was conducted at -50 KPa and 65 °C, while anhydrous cyclohexane was continuously supplemented until the distilled cyclohexane had no obvious water phase (with a water content below 100 ppm), and the solvent azeotropy-based dehydration was stopped. This process required about 2 h.

A sucrose content of a resulting solution reaction system was calculated to be about 20 wt%. The benzoic anhydride was added dropwise at a temperature lower than 10 °C with a ratio of the mass of the sucrose to the molar of benzoic anhydride being 1.0:1.05 to conduct acylation. After acylation at a temperature lower than 10 °C for 2 h, water was added with a ratio of the volume of the water to the total volume of a reaction solution being 0.25:1 to quench the reaction. Cyclohexane was added with a ratio of the volume of cyclohexane to the total volume of an obtained solution reaction system being 1:1, so as to extract the benzoyloxy-triphenyltin, and an obtained sucrose-6-benzoate solution was analyzed by HPLC. The product distribution was shown in Table 1.

### Comparative Example 1

150 g of sucrose and 600 mL of DMF were added to a 1000 mL four-neck round-bottom flask equipped with a mechanical stirrer, a thermometer, and a condenser having a water separator, and heated to 90 °C for dissolution. An obtained solution was cooled to 60 °C, and 112.5 g of di(butyl)tin oxide and 150 mL of cyclohexane were added thereto. The resulting mixture was heated to 90 °C, and an amount of the cyclohexane was adjusted to reflux and separate water. After reacting for 5 h, a reaction solution was cooled to 5 °C and 47.3 g of acetic anhydride was added dropwise within half an hour, and the resulting mixture was heated to room temperature, and stirred for another 6 h. 50 mL of water was added thereto, and a mixed solution was stirred for half an hour. Extractions were conducted three times with 200 mL of cyclohexane. 200 mL of cyclohexane was added to the extraction solution, and the resulting mixture was refluxed for 5 h for dehydration. Cyclohexane was finally distilled out, and a resulting solution was cooled to room temperature to obtain a DMF solution of sucrose-6-acetate. HPLC analysis shows that in the solution, sucrose-6-acetate has a purity of 95%, such that the solution could be directly used for the next step of chlorination. The product distribution was analyzed by HPLC as shown in Table 1.

**Table 1 Product distribution when mono-tin organic compounds was used in the synthesis of sucrose-6-carboxylates**

| | Example 5 | Example 6 | Example 7 | Example 8 | Comparative Example 1 |
|---|---|---|---|---|---|
| Catalyst type | Catalyst 1A | Catalyst 1B | Catalyst 1C | Catalyst 1D | Dibutyltin oxide |
| Sucrose-6-carboxylate content (%, normalized) | 17.53 (89.9%) | 18.51 (92.1%) | 17.83 (90.5%) | 19.48 (91.9%) | 19.25 (87.5) |
| Diacid ester (%, normalized) | 1.68 (8.6%) | 1.45 (7.2%) | 1.50 (7.6%) | 1.44 (6.8%) | 2.46 (11.2) |
| Sucrose (%, normalized) | 0.05 (0.24%) | 0.06 (0.30%) | 0.04 (0.21%) | 0.06 (0.32%) | 0.08 (0.36) |
| Catalyst recovery rate, % | 98% | 99% | 98% | 99% | 95% |

As shown in Table 1, for product distributions of Examples 5 to 8 and Comparative Example 1, the yields of sucrose-6-carboxylate in Examples 5 to 8 each are significantly higher than that of Comparative Example 1, indicating that a high yield are achieved in Examples 5-8. The content of diacid ester and sucrose was significantly lower than that of Comparative Example 1, indicating that high conversion rates of raw materials are achieved in Examples 5 to 8. Moreover, the catalyst recovery rate could reach not less than 98% in Examples 5 to 8, which was significantly higher than 95% in Comparative Example 1. This indicated that the mono-tin organic compound catalyst according to the present disclosure could also achieve a high recovery effect under the premise of ensuring a catalytic effect, which greatly reduces the production cost of sucralose.

In summary, the present disclosure has beneficial effects as follows: a mono-tin organic compound is provided, which can be used for the synthesis of sucrose-6-carboxylate by an organotin method. During the synthesis of the sucrose-6-carboxylate, the use of the mono-tin organic compound enables accurate dosing, and results in improved catalyst recovery rate, and reduced occurrence of subsequent chlorination side reactions, and moreover rapid reaction, a low energy consumption, and a higher yield per unit volume catalyst. Also, the use of mono-tin organic compound results in saved floor space, simplified process, and reduced waste discharge and treatment, which is environmentally-friendly.

## Claims

1. Use of a mono-tin organic compound in the synthesis of sucrose-6-carboxylate, the mono-tin organic compound having a structure shown in formula (1): 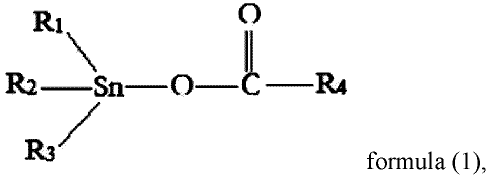
wherein R₁, R₂, and R₃ each are independently selected from the group consisting of C₁ to C₈ linear or branched saturated alkyl, C₂ to C₈ linear or branched unsaturated alkyl, C₃ to C₈ substituted or unsubstituted saturated cycloalkyl, C₃ to C₈ substituted or unsubstituted unsaturated cycloalkyl, and C₆ to C₁₂ aryl or substituted aryl; and
R₄ is selected from the group consisting of C₁ to C₆ linear or branched saturated alkyl and C₆ to C₁₂ aryl or substituted aryl; and
wherein the sucrose-6-carboxylate is synthesized by a method comprising:
using the mono-tin organic compound as a catalyst and conducting a dehydration esterification on sucrose in a polar aprotic solvent to obtain a sucrose organotin ester-containing solution.

2. The use as claimed in claim 1, wherein the R₁, the R₂, and the R₃ each are C₁ to C₆ linear or branched saturated alkyl, preferably n-butyl; and
the R₄ is methyl or phenyl.

3. The use as claimed in claim 1 or 2, wherein the dehydration esterification is conducted by any one of solvent azeotropy, negative-pressure distillation, atomization drying dehydration, and vacuum tower dehydration; and
the dehydration esterification is conducted at a temperature of 60 °C to 120 °C, preferably 70 °C to 100 °C and a pressure of 0.01 kPa to 50 kPa, preferably 0.5 kPa to 20 kPa for 0.1 min to 8 h, preferably 1 min to 1 h.

4. The use as claimed in claim 1 or 2, wherein the polar aprotic solvent is one or more selected from the group consisting of dimethyl sulfoxide, N-methylpyrrolidone, N,N-dimethylacetamide, hexamethylphosphoramide, and N,N-dimethylformamide, preferably N,N-dimethylformamide;
a ratio of a volume of the polar aprotic solvent to a mass of the sucrose is in a range of (2-20) mL: 1 g, preferably (3-10) mL: 1 g, and more preferably (4-8) mL: 1 g; and
a molar ratio of the mono-tin organic compound to the sucrose is in a range of 0.3 : 1 to 3 : 1, preferably 0.5 : 1 to 2.0 : 1, and more preferably 0.9 : 1-1.2 : 1.

5. The use as claimed in claim 1 or 2, wherein the method further comprises: cooling the sucrose organotin ester-containing solution, and conducting an acylation with an acylating agent to obtain the sucrose-6-carboxylate;
the acylation is conducted at a temperature of 0 °C to 50 °C, preferably 5 °C to 20 °C; and
a mass ratio of the acylating agent to the sucrose is in a range of 0.6 : 1 to 3.0 : 1, preferably 0.8 : 1 to 1.5 : 1.

## Patentansprüche

1. Verwendung einer organischen Mono-Zinn-Verbindung bei der Synthese von Saccharose-6-Carboxylat, die organische Mono-Zinn-Verbindung weist eine in Formel (1) gezeigte Struktur auf: 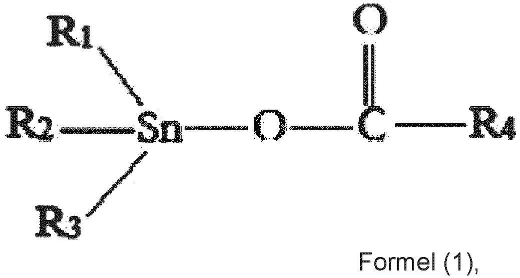
worin R₁, R₂ und R₃ jeweils unabhängig voneinander ausgewählt sind aus der Gruppe bestehend aus linearem oder verzweigtem gesättigtem C₁ bis C₈ Alkyl, linearem oder verzweigtem ungesättigtem C₂ bis C₈ Alkyl, substituiertem oder unsubstituiertem gesättigtem C₃ bis C₈ Cycloalkyl, substituiertem oder unsubstituiertem ungesättigtem C₃ bis C₈ Cycloalkyl und C₆ bis C₁₂ Aryl oder substituiertem Aryl; und
R₄ ausgewählt ist aus der Gruppe bestehend aus linearem oder verzweigtem gesättigtem C₁ bis C₆ Alkyl und C₆ bis C₁₂ Aryl oder substituiertem Aryl; und
wobei das Saccharose-6-Carboxylat durch ein Verfahren synthetisiert wird, das Folgendes umfasst:
Verwendung der organischen Mono-Zinn-Verbindung als Katalysator und Durchführung einer Dehydratations-Veresterung an Saccharose in einem polaren aprotischen Lösungsmittel, um eine organische Saccharose-Zinn-Ester-haltige Lösung zu erhalten.

2. Die Verwendung wie in Anspruch 1 beansprucht, worin der R₁, der R₂ und der R₃ jeweils lineares oder verzweigtes gesättigtes C₁ bis C₆ Alkyl sind, vorzugsweise n-Butyl; und
der R₄ ist Methyl oder Phenyl.

3. Die Verwendung wie in Anspruch 1 oder 2 beansprucht, worin die Dehydratations-Veresterung durch eine der folgenden Methoden durchgeführt wird: Lösungsmittel-Azeotropie, Unterdruck-Destillation, Zerstäubung Trocknen Dehydratation, und Vakuumturm-Dehydratation; und
die Dehydratations-Veresterung wird bei einer Temperatur von 60 °C bis 120 °C, vorzugsweise 70 °C bis 100 °C, und einem Druck von 0,01 kPa bis 50 kPa, vorzugsweise 0,5 kPa bis 20 kPa, für 0,1 min bis 8 h, vorzugsweise 1 min bis 1 h, durchgeführt.

4. Die Verwendung wie in Anspruch 1 oder 2 beansprucht, wobei das polare aprotische Lösungsmittel eines oder mehrere ist, ausgewählt aus der Gruppe bestehend aus Dimethylsulfoxid, N-Methylpyrrolidon, N,N-Dimethylacetamid, Hexamethylphosphoramid und N,N-Dimethylformamid, vorzugsweise N,N-Dimethylformamid;
ein Verhältnis eines Volumens des polaren aprotischen Lösungsmittels zur Masse der Saccharose ist im Bereich von (2-20) mL: 1 g, vorzugsweise (3-10) mL: 1 g und besonders bevorzugt (4-8) mL: 1 g; und
ein molares Verhältnis der organischen Mono-Zinn-Verbindung zu der Saccharose ist im Bereich von 0,3 : 1 bis 3 : 1, vorzugsweise 0,5 : 1 bis 2,0 : 1 und besonders bevorzugt 0,9 : 1-1,2 : 1.

5. Die Verwendung wie in Anspruch 1 oder 2 beansprucht, worin das Verfahren weiterhin umfasst: Kühlen der organischen Saccharose-Zinn-Ester-haltigen Lösung, und Durchführen einer Acylierung mit einem Acylierungsmittel, um das Saccharose-6-carboxylat zu erhalten;
die Acylierung wird bei einer Temperatur von 0 °C bis 50 °C, vorzugsweise 5 °C bis 20 °C, durchgeführt; und
ein Masseverhältnis des Acylierungsmittels zu der Saccharose ist in einem Bereich von 0,6:1 bis 3,0:1, vorzugsweise 0,8:1 bis 1,5:1.

## Revendications

1. Utilisation d'un composé organique mono étain dans la synthèse du sucrose-6-carboxylate, le composé organique mono étain ayant une structure représentée par la formule (1): 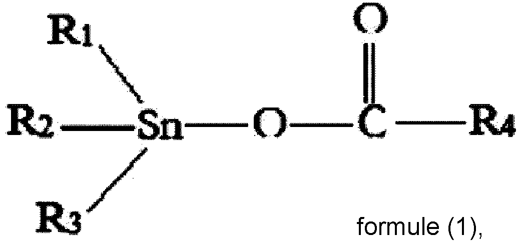
dans laquelle R₁, R₂ et R₃ sont chacun indépendamment choisis dans le groupe constitué par un alkyle saturé linéaire ou ramifié en C₁ à C₈, un alkyle insaturé linéaire ou ramifié en C₂ à C₈, un cycloalkyle saturé substitué ou non substitué en C₃ à C₈, un cycloalkyle insaturé substitué ou non substitué en C₃ à C₈ et un aryle ou un aryle substitué en C₆ à C₁₂; et
R₄ est choisi dans le groupe constitué d'alkyles saturés linéaires ou ramifiés en C₁ à C₆ et d'aryles en C₆ à C₁₂ ou d'aryles substitués; et
dans laquelle le sucrose-6-carboxylate est synthétisé par une méthode comprenant:
l'utilisation du composé organique mono étain comme catalyseur et la réalisation d'une estérification par déshydratation sur le saccharose dans un solvant aprotique polaire afin d'obtenir une solution contenant de l'ester organotine de saccharose.

2. L'utilisation selon la revendication 1, dans laquelle le R₁, le R₂ et le R₃ sont chacun des alkyles saturés linéaires ou ramifiés en C₁ à C₆, de préférence du n-butyle; et
le R₄ est méthyle ou phényle.

3. L'utilisation selon la revendication 1 ou 2, dans laquelle l'estérification de la déshydratation est réalisée par l'une quelconque des méthodes suivantes : azéotro-pie de solvant, distillation à pression négative, déshydratation de séchage par atomisation et déshydratation de tour à vide; et
l'estérification de déshydratation est réalisée à une température de 60 °C à 120 °C, de préférence 70 °C à 100 °C et une pression de 0,01 kPa à 50 kPa, de préférence 0,5 kPa à 20 kPa pendant 0,1 min à 8 h, de préférence 1 min à 1 h.

4. L'utilisation selon la revendication 1 ou 2, dans laquelle le solvant aprotique polaire est un ou plusieurs choisi dans le groupe constitué par le diméthylsulfoxyde, la N-méthylpyrrolidone, le N,N-diméthylacétamide, l'hexaméthylphosphoramide, et le N,N-diméthylformamide, de préférence le N,N- diméthylformamide;
un rapport entre le volume du solvant aprotique polaire et la masse de saccharose est de l'ordre de (2-20) mL: 1 g, de préférence (3-10) mL: 1 g et plus préférentiellement (4-8) mL: 1g; et
un rapport molaire entre le composé organique mono étain et le saccharose est de l'ordre de 0,3 : 1 à 3 : 1, de préférence 0,5 : 1 à 2,0 : 1, et plus préférentiellement 0,9 : 1-1,2 : 1.

5. L'utilisation selon la revendication 1 ou 2, dans laquelle le procédé comprend en outre: le refroidissement de la solution contenant de l'ester organotine de saccharose, et la réalisation d'une acylation avec un agent acylate pour obtenir le saccharose-6-carboxylate;
l'acylation est effectuée à une température de 0 °C à 50 °C, de préférence de 5 °C à 20 °C; et
un rapport massique de l'agent acylate sur le saccharose est compris dans une plage de 0,6: 1 à 3,0: 1, de préférence 0,8: 1 à 1,5: 1.
